# EUROPÄISCHE PATENTSCHRIFT

(11) **EP 1 851 552 B1**
(45) Veröffentlichungstag und Bekanntmachung des Hinweises auf die Patenterteilung: **23.04.2014**
(21) Anmeldenummer: 06706670.4
(22) Anmeldetag: 06.02.2006
(51) Int. Cl.: G01N 33/86, C12Q 1/56, G01N 33/49

(54) **METHODE ZUR DIFFERENZIERUNG VON FAKTOR XIII- GEGENÜBER FIBRINOGEN- MANGELZUSTÄNDEN UNTER EINSATZ THROMBELASTOGRAPHISCHER TECHNIKEN**
METHOD FOR DIFFERENTIATING A XIII FACTOR AND FIBRINOGEN DEFICIENCY STATES BY MEANS OF THROMBELASTOGRAPHIC ENGINEERING
PROCEDE DE DIFFERENCIATION DE FACTEUR XIII ET D'ETATS DE CARENCE EN FIBRINOGENE PAR L'INTERMEDIAIRE DE TECHNIQUES THROMBOELASTOGRAPHIQUES

(30) Priorität: 08.02.2005 DE 102005005824
(43) Veröffentlichungstag der Anmeldung: 07.11.2007
(60) Teilanmeldung: 12160723.8
(73) Patentinhaber: CSL Behring GmbH, 35041 Marburg (DE)
(72) Erfinder: WRABETZ, Erhardt, 65239 Hochheim (DE); METZNER, Hubert, 35041 Marburg (DE); KORTE, Wolfgang, 9010 St. Gallen (CH)
(74) Vertreter: Lauppe, Hans Friedrich
(86) Internationale Anmeldenummer: PCT/EP2006/001015
(87) Internationale Veröffentlichungsnummer: WO 2006/084648

(56) Entgegenhaltungen:
- WO-A-01/07070
- WO-A-2004/081579
- WO-A-2004/092742
- US-B1- 6 245 573
- CRAFT R M ET AL: "A NOVEL MODIFICATION OF THE THROMBELASTOGRAPH ASSAY (TEG(R)) WHICH ISOLATES PLATELET" ANESTHESIOLOGY, AMERICAN SOCIETY OF ANESTHESIOLOGISTS, PHILADELPHIA, PA,, US, Nr. 2003, 15. Oktober 2003 (2003-10-15), Seite ABSTRACTNO148, XP009066529 ISSN: 0003-3022
- KATORI N ET AL: "A NOVEL METHOD TO ASSESS PLATELET INHIBITION BY EPTIFIBATIDE WITH THROMBELASTOGRAPH" ANESTHESIA AND ANALGESIA, WILLIAMS AND WILKINS, BALTIMORE, MD, US, Bd. 99, Nr. 6, Dezember 2004 (2004-12), Seiten 1794-1799, XP009066521 ISSN: 0003-2999
- NIELSEN VANCE G ET AL: "The impact of factor XIII on coagulation kinetics and clot strength determined by thrombelastography" ANESTHESIA & ANALGESIA, Bd. 99, Nr. 1, Juli 2004 (2004-07), Seiten 120-123, XP009066431 ISSN: 0003-2999 in der Anmeldung erwähnt
- NIELSEN V G ET AL: "Effects of coagulation factor deficiency on plasma coagulation kinetics determined via thrombelastography(R): critical roles of fibrinogen and factors II, VII, X and XII" ACTA ANAESTHESIOLOGICA SCANDINAVICA, Bd. 49, Nr. 2, Februar 2005 (2005-02), Seiten 222-231, XP002383695 ISSN: 0001-5172

## Beschreibung

### 1. Einleitung

Die Erfindung betrifft eine Methode zur Differenzierung von Faktor XIII Mangelzuständen gegenüber Fibrinogen Mangelzuständen durch gleichzeitige Bestimmung beider potentieller Mangelzustände aus einer Probe sowie deren Vergleich,
einerseits,
- durch Vergleich von bestimmten TEG Parametern in Anwesenheit, mit bestimmten TEG Parametern in Abwesenheit von Inhibitoren von Faktor XIII
   oder
- durch Vergleich von bestimmten TEG Parametern bei Zugabe mit denen ohne Zugabe von Faktor XIII
   sowie andererseits
- durch Vergleich von bestimmten TEG Parametern in Anwesenheit von Fibrinogen Aktivatoren, mit bestimmten TEG Parametern in Anwesenheit von Aktivatoren von Fibrinogen in Normal-Blut oder -Plasmen.

### 2. Darstellung des Standes der Technik

Die Thrombelastographie (TEG) ist eine diagnostische Methode, die mechanisch die Gerinnseientstehung oder -Auflösung in einem oszillierenden System untersucht. Dabei wird entweder ein Gefäß (Cup) oszillierend um einen Messstab (Pin) bewegt (herkömmliche TEG) oder aber das Gefäß steht fest und der Pin wird in oszillierende Rotationsbewegungen gebracht (ROTEG bzw. ROTEM). Die zwischen Cup und Pin auftretenden mechanischen Kräfte werden aufgezeichnet. Sobald das Blut oder Plasma zur Gerinnung kommt, tritt eine Abweichung zum anfänglichen Messsignal auf. Beide Ausführungen sollen hier als TEG bezeichnet werden.

Die TEG wird zur Untersuchung von Blut oder Plasma eingesetzt, um gerinnungsrelevante Parameter (TEG Parameter), wie die Zeitspanne bis zum Erreichen einer ersten signifikanten Clot-Bildung mit einer Amplitude von 2mm (Gerinnungs- oder Reaktionszeit r), die Zeitspanne bis zum Erreichen einer Clot-Stärke von einer Amplitude von 20 mm (k-Wert), die Geschwindigkeit, mit der das Gerinnsel gebildet wird (Winkel Alpha), die mechanischen Eigenschaften des Gerinnsels bei maximaler Amplitude (MA) oder zu einem anderen beliebigen Zeitpunkt, die Zeitspanne bis zur MA (TMA), oder die Zeitspanne bis die Gerinnselfestigkeit aufgrund von Fibrinolyse auf einen bestimmten Wert wieder abgefallen ist, zu bestimmen. Klinisch wird die TEG u.a. zur Beurteilung der Koagulopathie beispielsweise in der Herzchirurgie, bei Lebertransplantationen, großer Abdominalchirurgie und als quasi Bedside-Test im perioperativen Gerinnungsmanagement als diagnostische Maßnahme eingesetzt (Kettner SC et al. (1999), Anesth Analg; 89: 580-584; Shore-Lesserson L et al. (1999), Anesth Analg; 88: 312-319et al.; Harding SA et al. (1997), Br J Anaesth; 78: 175-179; Kettner SC et al. (1998), Anesth Analg; 86: 691-695, Pivalizza EG et al. (1998), J Cardiothorac Vasc Anesth; 12: 305-308., Mahla E et al. (2001), Anest Analg; 92: 572-577; Calatzis AN et al. (1996), Eur Surg Res; 28: S1 (89), Mahla E et al. (2001), Anesth Analg; 92 (3): 572-577).

Die TEG steht ergänzend zur Standard Gerinnungsdiagnostik (u.a. Quick, aPTT, Thrombozytenzahl, AT III, Fibrinogen, D-Dimere, Blutungszeit), die zur Ermittlung der Werte zeitaufwendiger ist, für eine schnelle Orientierung über Blutungstendenzen zur Verfügung. Dies ist besonders dann von Bedeutung, wenn Koagulopathien im Rahmen von extensiven chirurgischen Eingriffen oder nach Polytraumata auftreten, da schwere Störungen der Hämostase rasch zur Entwicklung von sekundären Gewebeschäden führen können und oft resistent sind gegenüber einer konventionellen hämostatischen Therapie auf der Basis einer zeitaufwendigeren Standard-Gerinnungsdiagnostik.

Die genannten TEG Parameter werden durch eine Reihe von Faktoren beeinflusst, die im Folgenden als thrombelastographisch relevante Faktoren bezeichnet werden. Thrombelastographisch relevante Faktoren sind vor allem Fibrinogen-, Faktor XIII- und Blutplättchen-Spiegel sowie Komponenten des fibrinolytischen Systems wie Plasmin, Plasmin-Aktivatoren und Plasmin-Inhibitoren. So korreliert beispielsweise Fibrinogen als Substrat der Gerinnung mit der Clot-Stabilität. Dies kann im Thrombelastogramm eindeutig gezeigt werden. Darüber hinaus moduliert der Faktor XIII über die Vernetzung des aus dem Fibrinogen gebildeten Fibrins ebenfalls die mechanischen Eigenschaften des Clots und schränkt dessen Lyse ein (P. Lauer et al. (2002), Thromb Haemost; 88:967-974). Beide Effekte sind in der Thrombelastographie bislang nicht eindeutig voneinander trennbar, da sie zentrale Messgrößen eines Thrombelastogrammes gleichermaßen beeinflussen. Sowohl Fibrinogen als auch Faktor XIII beeinflussen die Parameter Gerinnungszeit (r), maximale Clot-Festigkeit, Winkel Alpha als Maß für die Geschwindigkeit der Clot-Bildung und die Lysezeit des Clots signifikant (Nielsen VG et al. (2004), Anesth Analg; 99: 120-123). Da bei erworbenen Mangelzuständen, beispielsweise bei Polytraumata oder größeren chirurgischen Eingriffen der Fibrinogen- und der Faktor XIII-Spiegel nicht notwendigerweise gleichermaßen abfallen, ist bislang eine gezielte therapeutische Intervention nicht eindeutig zugunsten der einen oder anderen Komponente auf Basis des Thrombelastogramms möglich. Es wäre daher wünschenswert, wenn eine Methode zur Verfügung stünde, die es erlauben würde, auf Basis der schnellen TEG Diagnostik eine Aussage darüber treffen zu können, ob eine Blutungsneigung auf einem Faktor XIII- und/oder auf einem Fibrinogen-Mangel beruht.

WO2004/092742 beschreibt ein Verfahren zur Messung eines Thrombinmangels. Dabei wird den Proben Heparin als indirektes Antithrombin Antikoagulant zugegeben.

WO0107070 offenbart ein Verfahren zur Messung der Aktivität von Gerinnungsfaktoren, wobei den Proben Inhibitoren gegen verschiedene Gerinnungsfaktoren zugegeben werden und die Gerinnungszeit gemessen wird. Die Methode dient dem Auffinden von hyperkoagulativen Zuständen.

WO2004081579 offenbart die Verwendung der Thrombelastographie zur Verfolgung verschiedener thrombelastographischer Parameter bei der Plättchenhemmung.

US 6,245,573 befasst sich mit dem Einfluss von Metallionen auf die Viskosität und die Gerinnungsaktivität von Blut

Craft et al. (American Society of Anesthesiologists, Philadelphia, PA, US Nr. 2003, 15.Okt. 2003, Abstract Nr. 148)

Katori et al. (Anesthesia and Analgesia, Bd. 99, Nr. 6 (2004) beschreibt ein mittels Batroxobin modifizierte Thrombelastographie zum Nachweis einer Eptifibatide induzierten Plättchenhemmung.

Nielsen et al. (Acta Anaeasthesiologica Scandinavia, Bd. 49, Nr. 2 Seiten 222-231 (2005) befasst sich mit der Rolle von Fibrinogen bei thrombelastographischen Messungen. Es zeigt sich eine klare Abhängigkeit der Thrombelastographie von der Fibrinogen Konzentration.

Estner (Dissertation eingereicht am 23.4.2001 bei der Technischen Universität München) untersucht die Rolle von Faktor XIII auf die Gerinnselbildung und Gerinnselfestigkeit vor und nach herzchirurgischen Eingriffen mit Hilfe der Thrombelastographie.

Wilmer et al. (Hämatoserologie 1, (2002)) beschreibt die Verwendung verschiedener Methoden, darunter auch der Thrombelastographie für die Bestimmung der Gerinnselbildung und der Gerinnselqualität.

Tyler et al. ((1969) untersucht den Einfluss der Quervernetzung von Fibrin auf thrombelastographische Parameter.

### 3. Darstellung von Aufgabe und Lösung

Den vorliegenden Untersuchungen lag die Aufgabe zu Grunde, mittels thrombelastographischer Techniken eine eindeutige Differenzierung der Einflussgrößen Faktor XIII und Fibrinogen bei Hämostasestörungen zu ermöglichen und ihren jeweiligen Anteil an der Hämostasestörung zu bewerten, um dadurch eine gezielte therapeutische Intervention durch Substitution des fehlenden Faktors zu ermöglichen.

Gelöst wurde die Aufgabe dadurch, dass ein Verfahren entwickelt wurde, das zur Differenzierung von Faktor XIII Mangelzuständen gegenüber Fibrinogen Mangelzuständen dient durch gleichzeitige Bestimmung beider potentieller Mangelzustände aus einer Probe sowie deren Vergleich.

Insbesondere kann der Gehalt der thrombelastographischen Faktoren Faktor XIII und Fibrinogen differenziert werden, indem die Messung mit und ohne Inhibitoren für Faktor XIII und/oder mit und ohne Zugabe von Faktor XIII und/oder durch Zugabe von Aktivatoren von Fibrinogen durchgeführt wird. Durch den Vergleich der TEG Parameter dieser Ansätze lässt sich der Einfluss der Einzelfaktoren bestimmen und der Grad der notwendigen Substitution der Einzelkomponenten zur Erreichung einer stabilen Hämostase ermitteln. Hierdurch können beispielsweise intraoperative massive Blutungen noch gezielter und schneller therapiert werden und Risiken für postoperative Hämostasestörungen, und unter Umständen dadurch bedingte Wundheilungsstörungen, erkannt und ebenfalls durch Substitution der Einzelkomponenten gezielt vermieden werden. Weitere Komponenten, die die TEG Parameter beeinflussen, wie Thrombozytenzahl und -aktivität oder fibrinolytische Aktivität, können durch bekannte Verfahren unterdrückt werden.

### 4. Detaillierte Beschreibung der Erfindung und verschiedener Ausführungsformen

Durch Vergleich der TEG Parameter, wie beispielsweise der Reaktionszeit (r), der maximalen Amplitude (MA), der Zeit bis zum Erreichen der maximalen Amplitude (TMA) oder des Winkels Alpha in An- oder Abwesenheit von Faktor XIII Inhibitoren, kann man zwischen einem Faktor XIII-Mangel und anderen Mangelzuständen als Ursache für Hämostasestörungen differenzieren.

Zur Hemmung des Faktor XIII können beispielsweise Antikörper gegen Faktor XIII, peptidische Inhibitoren wie Tridegin (Finney S. et al. (1997), Biochem J. 324: 797-805) oder niedermolekulare Inhibitoren des Faktor XIII, wie beispielsweise Putrescin, Dansylcadaverin o.a. (Prasa D. et al. (2002), Hämostaseologie 22: 29-33) verwendet werden. Der Faktor XIII Inhibitor bzw. die Konzentration des Faktor XIII Inhibitors ist bevorzugt so zu wählen, dass die Faktor XIII Aktivität in der untersuchten Probe spezifisch und vollständig inhibiert wird.

Je geringer der Faktor XIII-Spiegel bei einem Patienten ist, desto geringer wird dabei der Unterschied zwischen dem Wert eines TEG Parameters, den man in Anwesenheit und dem, den man in Abwesenheit eines Faktor XIII-Inhibitors misst. Die Auswertung der Differenz der TEG Parameter in An- und Abwesenheit eines Faktor XIII Inhibitors lässt darauf schließen, ob ein leichter, mittlerer oder schwerer Faktor XIII Mangel vorliegt. Durch Aufstocken einer von einem Patienten mit Faktor XIII Defizienz gewonnenen Vollblutprobe mit unterschiedlichen Mengen an Faktor XIII und durch Vergleich des Thrombelastogramms in An- und Abwesenheit eines Inhibitors von Faktor XIII in diesen Proben lässt sich der Effekt auch in Form einer Standardkurve darstellen. Eine solchermaßen gewonnene Kurve erlaubt es, aus der Differenz eines TEG Parameters in An- und Abwesenheit eines Faktor XIII-Inhibitors den Faktor XIII-Spiegel in einer Patientenprobe zu bestimmen. Bevorzugt werden entsprechende Standardkurven bei unterschiedlichen Fibrinogen-Gehalten erstellt.

Durch Bezug der Differenz der Patientenprobe in An- und Abwesenheit eines Faktor XIII Inhibitors auf das Verhältnis des TEG Parameters (mit Inhibitorzusatz) des Patientenplasmas zu einer Kontrollprobe ergibt sich auch die Möglichkeit, die Messdifferenz zu relativieren und somit unterschiedliche Fibrinogenspiegel zu berücksichtigen, z.B. durch Bildung des folgenden Terms im Falle der maximalen Amplitude: (MA_{Probe ohne Inh.} - MA_{Probe mit Inh}.) MA_{Kontrolle mit Inh} / MA_{Probe mit Inh}.).
Analoge Standardkurven können auch mit plättchenarmem und plättchenreichem Plasma erhalten werden. Unterhalb eines Wertes von 70% des Normalwerts von Faktor XIII in Plasma spricht man von Faktor XIII Mangelzuständen, die therapiert werden sollten (T. Muto et al. (1997), Biomed. Progress 10: 16-19).

Ein Faktor XIII-Mangel lässt sich alternativ auch durch eine umgekehrte Vorgehensweise detektieren, indem man eine Probe mit und ohne Zusatz von Faktor XIII in der TEG vergleicht. Ein geringer Unterschied der TEG Parameter deutet hier, umgekehrt wie bei Einsatz von Faktor XIII-Inhibitoren, auf einen Normalwert des Faktor XIII in der Probe hin, während große Unterschiede auf einen schweren Faktor XIII Mangel hinweisen. Wie oben beschrieben, lassen sich auf Basis von Faktor XIII defizientem Vollblut, bzw. Plasma auch für diese Vorgehensweise Standardkurven erstellen, die eine genauere Diagnostik erlauben. Bei Zugabe von Faktor XIII wird mindestens eine Menge zugegeben, die 100% Faktor XIII auch bei einem völligen Faktor XIII Mangel einstellen würde. Bevorzugt setzt man sogar noch höhere F XIII Mengen ein, gegenüber denen der in der Probe enthaltene F XIII vernachlässigbar ist.

Um eine Differentialdiagnostik gegenüber dem die TEG Parameter ebenfalls stark beeinflussenden Fibrinogen-Spiegel zu verbessern, ist ein weiterer Aspekt der vorliegenden Erfindung, die Bestimmung des Fibrinogen-Spiegels im Blut des Patienten mit Hilfe thrombelastographischer Parameter.

Eine Methode, einen möglichen Fibrinogen-Mangel zu evaluieren, besteht in der Behandlung einer Vollblutprobe, bzw. eines plättchenarmen oder plättchenreichen Plasmas mit Proteasen, die Fibrinogen aktivieren, gegenüber Faktor XIII aber nicht reaktiv sind. So kann beispielsweise durch die Verwendung von Batroxobin, einer aus Schlangengift isolierten Protease, das Fibrinogen der Probe in Fibrin überführt werden (ohne Quervernetzung durch Faktor XIII) und im Vergleich zu einer Normal-Kontrolle (Blut oder Plasma gesunder Spender) eine Fibrinogen-Erniedrigung detektiert werden. Es kann dabei vorteilhaft sein, in Anwesenheit von Hirudin zu arbeiten, um eine Faktor XIII-Aktivierung im Verlaufe der Messung zu vermeiden. Bei der Durchführung einer solchen TEG-Untersuchung sollte die Proteasen-Menge, die zugesetzt wird, ausreichen, um das Fibrinogen innerhalb weniger Minuten zu aktivieren und unter Ausbildung eines Gerinnsels zu polymerisieren. Im Falle von Batroxobin sollte das Fibrinogen innerhalb weniger Minuten zu des-AA-Fibrinogen umgesetzt werden, das zu einem Gerinnsel polymerisiert. Je größer der Unterschied zum Normalwert des untersuchten TEG-Parameters ist, desto größer ist der Fibrinogenmangel in der Patientenprobe. Die Aktivierung von Fibrinogen findet vorteilhafter Weise in Anwesenheit von die Aktivierung von Faktor XIII hemmenden Faktoren statt, wie beispielsweise in Gegenwart von Thrombin-Inhibitoren wie z.B. Hirudin.

Da Thrombozyten ebenfalls einen Einfluss auf die Clot-Eigenachaften und damit auf die TEG Parameter in Vollblut haben können, ist die bevorzugte Durchführung der Differentialdiagnostik bei gleichzeitigem Ausschluss von Thrombozyteneffekten ein weiterer Aspekt dieser Erfindung. Beispielsweise ist die Verwendung von plättchenarmem Plasma möglich, da die Differenzierung von Fibrinogen und Faktor XIII prinzipiell sowohl in Vollblut als auch in Plasma möglich ist. Im Falle von Plasma sollte dann zur Initiierung der Gerinnung entweder ein Oberflächenreagenz wie beispielsweise aPTT Reagenz oder Gewebefaktorreagenz verwendet werden. Alternativ kann bei Verwendung von plättchenreichem Plasma oder von Vollblut die Ermittlung der TEG Parameter in Anwesenheit von Plättchenantagonisten, wie beispielsweise Cytochalasin und/oder Abciximab unter Ausschluss von Thrombozyteneffekten durchgeführt werden. Eine besonders bevorzugte Ausführungsform ist die Ausschaltung der Plättcheneffekte durch eine Kombination von Cytochalasin D und Abciximab (Lang et al.; J Thromb Haemost, 2004; 2(1): 147-53).

Da das thrombolytische System ebenfalls einen Einfluss auf die Clot-Eigenschaften und damit auf die TEG Parameter haben kann, ist zum Ausschluss von Einflüssen des thrombolytischen Systems die Hemmung der Komponenten des thrombolytischen Systems vorteilhaft. Ein weiterer Aspekt dieser Erfindung ist daher die Durchführung der beschriebenen Differentialdiagnostik bei gleichzeitiger Hemmung von thrombolytischen Aktivitäten wie z.B. von Plasmin oder Plasmin-Aktivatoren bzw. bei gleichzeitiger Aktivierung von Plasmin-Inhibitoren. Eine bevorzugte Ausführungsform ist dabei die Durchführung der TEG in Gegenwart von Aprotinin, α2-Antiplasmin oder ähnlichen Inhibitoren oder auch niedermolekularen Inhibitoren.

Eine besonders bevorzugte Ausführungsform ist die Durchführung der TEG bei gleichzeitigem Ausschluss des Plättcheneinflusses sowie auch der Ausschaltung des thrombolytischen Systems.

Die beschriebenen Verfahren erlauben es, im thrombelastographischen System den Effekt der Plättchen und des thrombolytischen Systems zu eliminieren und eine Differenzierung zwischen einem Faktor XIII- und einem Fibrinogen-Mangel durchzuführen. Der besondere Vorteil liegt dabei darin, dass zeit- und patientennah mittels TEG der Faktor XIII- und der Fibrinogen-Spiegel ermittelt werden kann und das diagnostische Ergebnis somit direkt therapeutisch umgesetzt werden kann.
Die Verwendung von Faktor XIII Inhibitoren ermöglicht es auch, durch TEG in An- und Abwesenheit von Plättchenantagonisten oder Fibrinolyseinhibitoren Faktor XIII als Einflussfaktor auf diese Messungen auszuschalten und somit Plättcheneffekte eindeutiger zu diagnostizieren.

Gegenstand der Erfindung ist eine Methode zur Differenzierung von Faktor XIII Mangelzuständen gegenüber Fibrinogen Mangelzuständen durch gleichzeitige Bestimmung beider potentieller Mangelzustände aus einer Probe sowie deren Vergleich,
einerseits,
- durch Vergleich von bestimmten TEG Parametern in Anwesenheit, mit bestimmten TEG Parametern in Abwesenheit von Inhibitoren von Faktor XIII
   oder
- durch Vergleich von bestimmten TEG Parametern bei Zugabe mit denen ohne Zugabe von Faktor XIII
   sowie andererseits
- durch Vergleich von bestimmten TEG Parametern in Anwesenheit von Fibrinogen Aktivatoren, mit bestimmten TEG Parametern in Anwesenheit von Aktivatoren von Fibrinogen in Normal-Blut oder -Plasmen.

Durch Mehrkanalmessungen ist es möglich, eine differenzierte Aussage über den Substitutionsbedarf an Faktor XIII und/oder Fibrinogen zu erhalten.

In einer Ausführungsform eines diagnostischen Kits sind dabei die Reagenzien bereits in den TEG-Cups vorgelegt.

### 5. Beispiele

### 1. Hemmung von Faktor XIII in Plasma durch Zusatz von Faktor XIII-Antikörpern

Um den Einfluss von Faktor XIII quantitativ zu ermitteln, wurde in einem Experiment Standardhumanplasma ohne bzw. mit Zusatz von Faktor XIII Inhibitoren (Anti-Faktor XIII IgG Präparation) mittels TEG analysiert. Die Gerinnungsreaktion wurde durch Zusatz eines aPTT Reagenzes beschleunigt. Der Testansatz enthielt: NaCl-Lsg. oder anti-Faktor XIII IgG Präparation in unterschiedlichen Verdünnungen (30 µL), Pathromtin SL (50 µL, Dade Behring), Standard Humanplasma (200 µL) und 200 mM CaCl2-Lsg (20 µL). Bei 37°C wurden die Reagenzien in das Cup pipettiert und die TEG Messung an Geräten der Firma Haemoscope gestartet. Folgende Messparameter wurden ausgewertet: R, Winkel alpha, Maximalamplitude und Zeit bis zum Erreichen der Maximalamplitude.

**Tabelle 1: Hemmung von Faktor XIII in Plasma durch Zusatz von Faktor-XIII Antikörpern**

| | | **R (sec)** | **Angle (°)** | **MA (mm)** | **TMA (sec)** | |
|---|---|---|---|---|---|---|
| **Kontrolle (ohne F XIII AK)** | | **133,8** | **70,7** | **16,7** | **721,3** | Mittelwerte aus n=8 |
| **Anti FXIII A IgG Präp.** | **1:3** | **217,5** | **53,3** | **7,1** | **272,5** | n=2 |
| | **1:10** | **157,5** | **61,7** | **9,9** | **382,5** | n=2 |
| | **1:30** | **152,5** | **67,5** | **15,1** | **605,0** | n=2 |
| | **1:100** | **120,0** | **69,2** | **16,3** | **681,5** | n=2 |

Die Ergebnisse zeigen, dass sich die Faktor XIII-Inhibition deutlich auf die gemessenen TEG Parameter auswirkt. Entsprechend würde ein Vergleichsansatz (+/-Faktor XIII Inhibitor) bei einer Patientenprobe mit reduzierter maximaler Amplitude eine Abschätzung ermöglichen, ob noch eine ausreichende Faktor XIII-Menge in der Probe vorhandenen ist oder ob diese signifikant reduziert ist (bei reduziertem Faktor XIII-Spiegel würde der Effekt der Faktor XIII-Inhibitor Zugabe geringer ausfallen als bei normalem Faktor XIII-Spiegel).

### 2. Untersuchung von Plasmen und Vollblut durch parallele Messungen in An-und Abwesenheit von niedermolekularen Faktor XIII-Inhibitoren

Zur quantitativen Bestimmung des Einflusses von Faktor XIII wurde Standardhumanplasma ohne bzw. mit Zusatz niedermolekularer Faktor XIII Inhibitoren mittels TEG analysiert. Die Gerinnungsreaktion wurde durch Zusatz eines Gewebefaktorreagenzes gestartet bzw. beschleunigt. Der Testansatz enthielt: 200 µL Plasma, 30 µL NaCl-Lsg. (Kontrolle) oder Inhibitor-Lösung in unterschiedlichen Verdünnungen, 50 µL Gewebefaktorreagenz (Thromborel S, Dade Behring) und 20 µL Calciumchlorid-Lösung (200 mMol/L). Bei 37°C wurden die Reagenzien in das Cup pipettiert und die TEG Messung an Geräten der Firma Haemoscope gestartet. Alternativ kann das Calciumchlorid auch bereits zu dem Gewebefaktorreagenz zugegeben und damit die Reaktion gestartet werden. Die Messparameter Reaktionszeit (R), Winkel alpha, Maximalamplitude (MA) und Zeit bis zum Erreichen der Maximalamplitude (TMA) wurden erfasst und ausgewertet.
Als F XIII Inhibitoren zur Differenzierung des F XIII Gehaltes wurden Putrescin, Histidin, Dansylcadaverin bzw. 1,3,4,5-Tetramethyl-2-[(2-oxopropyl)thio]-imidazoliumchlorid in unterschiedlichen Verdünnungen eingesetzt. Die hier mit Plasma durchgeführten Untersuchungen sind prinzipiell auch auf Vollblut übertragbar.

**Tabelle 2: Effekt von Putrescin, Monodansylcadaverin und Histamin auf die TEG Parameter bei Verwendung von Standardhumanplasma.**

| **Putrescin** | **R:** | **Angle:** | **MA:** | **TMA:** |
|---|---|---|---|---|
| Konzentration im Test: (µg/mL) | **sec** | ° | **mm** | **sec** |
| **1,07** | **22,5** | **73,1** | **77,2** | **452,5** |
| **3,21** | **17,5** | **73,7** | **15,9** | **277,5** |
| **10,7** | **20,0** | **73,4** | **18,7** | **672,5** |
| **32,1** | **25** | **73,0** | **16,9** | **585,0** |
| **107** | **15** | **73,0** | **16,5** | **505,0** |
| **321** | **20** | **72,8** | **15,8** | **400,0** |
| **1.071** | **25** | **69,5** | **13,6** | **405,0** |
| **3.214** | **32,5** | **72,6** | **13,9** | **327,5** |
| | | | | |

| **Monodansylcadaverin** | **R:** | **Angle:** | **MA:** | **TMA:** |
|---|---|---|---|---|
| Konzentration im Test: (µg/mL) | **sec** | **°** | **mm** | **sec** |
| **0,32** | **20,0** | **74,0** | **18,1** | **655,0** |
| **1,07** | **22,5** | **74,9** | **18,4** | **610,0** |
| **3,21** | **20,0** | **74,7** | **19,1** | **657,5** |
| **10,7** | **25,0** | **74,4** | **18,4** | **560,0** |
| **32,1** | **17,5** | **74,4** | **17,3** | **562,5** |
| **107** | **32,5** | **76,9** | **18,7** | **237,5** |
| **321** | **20,0** | **71,0** | **15,1** | **490,0** |
| **536** | **27,5** | **70,3** | **13,3** | **370,0** |
| | | | | |

| **Histamin** | **R:** | **Angle:** | **MA:** | **TMA:** |
|---|---|---|---|---|
| Konzentration im Test: (µg/mL) | **sec** | **°** | **mm** | **sec** |
| **1,07** | **20,0** | **73,4** | **17,8** | **592,5** |
| **3,21** | **22,5** | **75,0** | **17,9** | **527,5** |
| **10,7** | **20,0** | **74,8** | **19,2** | **622,5** |
| **32,1** | **22,0** | **73,9** | **17,2** | **472,5** |
| **107** | **17,5** | **73,5** | **16,9** | **462,5** |
| **321** | **20,0** | **72,7** | **15,5** | **430,0** |
| **1.071** | **15,0** | **70,3** | **14,0** | **492,5** |
| **3.214** | **25,0** | **71,9** | **13,0** | **182,5** |
| | | | | |

| **Kontrolle** | **R:** | **Angle:** | **MA:** | **TMA:** |
|---|---|---|---|---|
| **(physiol. NaCl Lösung)** | **sec** | **°** | **mm** | **sec** |
| | **25,6** | **75,6** | **19,7** | **572** |

**Tabelle 3: Effekt von 1,3,4,5-Tetramethyl-2-[(2-oxopropyl)thio]-imidazoliumchlorid auf die TEG Parameter).**

| **1,3,4,5-Tetramethyl-2-** | **R:** | **Angle:** | **MA:** | **TMA:** |
|---|---|---|---|---|
| **[(2-oxopropyl)thio]-** | | | | |
| **imidazolium chloride** | **sec** | **°** | **mm** | **sec** |
| Konzentration im Test (µg/mL) | | | | |
| **0,033** | **22,5** | **79,6** | **21,5** | **177,5** |
| **0,10** | **22,5** | **79,0** | **18,5** | **140,0** |
| **0,33** | **22,5** | **77,0** | **14,9** | **77,5** |
| **1,00** | **22,5** | **75,4** | **13,1** | **77,5** |
| **3,33** | **22,5** | **74,0** | **12,4** | **77,5** |
| | | | | |

| **Kontrolle** | | | | |
|---|---|---|---|---|
| **(physiol. NaCl Lösung)** | | | | |
| | **27,5** | **77,7** | **21,6** | **520,0** |

Es zeigt sich, dass die untersuchten Inhibitoren die Wirkung von F XIII inhibieren können und die relevanten TEG-Parameter wie z.B. maximale Amplitude (MA) signifikant auf den noch verfügbaren F XIII-Gehalt ansprechen. Auf diese Art und Weise kann man F XIII defiziente Plasmen identifizieren, da solche Plasmen den Effekt der F XIII Inhibitoren nicht oder nur eingeschränkt zeigen. Aus den obigen Untersuchungen ergeben sich als bevorzugte Konzentrationen an Inhibitoren diejenigen, bei denen die durch F XIII beeinflussten Parameter wie die maximale Amplitude MA nicht mehr deutlich abfallen oder ansteigen. Für 1,3,4,5-Tetramethyl-2-[(2-oxopropyl)thio]-imidazoliumchlorid ist dies z.B. eine Endkonzentration von 1 µg/mL, besonders bevorzugt von ca. 3 µg/mL oder höher.

### 3. Differentielle Untersuchung zur Diagnostik eines Mangels an Fibrinogen und Faktor XIII (Hypothetisches Beispiel)

Differentielle TEG-Analytik zur Abschätzung der gerinnungsrelevanten Restkapazität von Faktor XIII und Fibrinogen bzw. weiterer Faktoren. Es kann die gesamte Palette an Bedingungen untersucht werden oder eine Auswahl entsprechend der jeweiligen Problematik getroffen werden.

### Experimenteller Ansatz:

a) unveränderte Probe,
b) Probe in Anwesenheit von Faktor XIII Antikörpern,
c) Probe durch Batroxobin zur Gerinnung gebracht (z.B. in Anwesenheit von Hirudin)
d) Probe in Anwesenheit von Thrombozyten-Antagonisten (im Falle von Vollblut oder plättchenreichern Plasma)
e) Probe in Anwesenheit von Aprotinin
f) Probe in Anwesenheit von Aprotinin und Thromboyzten-Antagonisten (im Fall von Vollblut oder plättchenreichem Plasma)
g) Proben in Anwesenheit von F XIII-Inhibitor, Thrombozyten-Antagonisten und Fibrinolyseinhibitor (im Fall von Vollblut oder plättchenreichem Plasma)

Ein Vergleich erfolgt gegen historische TEG-Messungen mit Normalblut oder Normalplasma in An- bzw. Abwesenheit der entsprechenden Zusätze.

## Patentansprüche

1. Methode zur Differenzierung von Faktor XIII Mangelzuständen gegenüber Fibrinogen Mangelzuständen durch gleichzeitige Bestimmung beider potentieller Mangelzustände aus einer Probe sowie deren Vergleich,
einerseits,
- durch Vergleich von bestimmten TEG Parametern in Anwesenheit, mit bestimmten TEG Parametern in Abwesenheit von Inhibitoren von Faktor XIII
oder
- durch Vergleich von bestimmten TEG Parametern bei Zugabe mit denen ohne Zugabe von Faktor XIII
sowie andererseits
- durch Vergleich von bestimmten TEG Parametern in Anwesenheit von Fibrinogen Aktivatoren, mit bestimmten TEG Parametern in Anwesenheit von Aktivatoren von Fibrinogen in Normal-Blut oder -Plasmen.

2. Methode nach Anspruch 1, wobei eine Feststellung von Faktor XIII Mangelzuständen durch Messung, Auswertung und Vergleich von bestimmten TEG Parametern in Anwesenheit und Abwesenheit von Faktor XIII Inhibitoren erfolgt.

3. Methode nach Anspruch 1, wobei eine Feststellung von Faktor XIII Mangelzuständen durch Messung, Auswertung und Vergleich von bestimmten TEG Parametern bei Zugabe von Faktor XIII und ohne Zugabe von Faktor XIII erfolgt.

4. Methode nach Anspruch 1, wobei eine Feststellung von Fibrinogen Mangelzuständen durch Vergleich thrombelastographischer Parameter in Anwesenheit von Fibrinogen Aktivatoren mit TEG Normalwerten erhalten durch Verwendung von Fibrinogen Aktivatoren in Normal-Blut oder -Plasmen erfolgt, wobei die Fibrinogen Aktivatoren Proteasen sind, die Fibrinogen aktivieren, gegenüber Faktor XIII aber nicht reaktiv sind.

5. Methode gemäß Anspruch 1 zur Differenzierung von Faktor XIII Mangelzuständen gegenüber Fibrinogen Mangelzuständen unter Ausschluss des Einflusses von Blutplättchen durch Ermittlung der TEG Parameter bei Verwendung von plättchenarmem Plasma oder durch den Einsatz von Plättchen-Antagonisten bei Verwendung von Vollblut oder plättchenreichem Plasma.

6. Methode gemäß Anspruch 1 zur Differenzierung von Faktor XIII Mangelzuständen gegenüber Fibrinogen Mangelzuständen unter Ausschluss von Einflüssen des thrombolytischen Systems durch Ermittlung der TEG Parameter bei Verwendung von Inhibitoren des thrombolytischen Systems.

7. Methode gemäß Anspruch 1 zur Differenzierung von Faktor XIII Mangelzuständen gegenüber Fibrinogen Mangelzuständen bei Verwendung von plättchenarmem Plasma oder durch den Einsatz von Plättchen-Antagonisten bei Verwendung von Vollblut oder plättchenreichem Plasma sowie bei gleichzeitiger Hemmung des thrombolytischen Systems gemäß den Ansprüchen 5 und 6.

8. Verwendung einer Methode gemäß Ansprüchen 1 bis 7 als Grundlage für die Entscheidungsfindung über eine gezielte Substitution von Faktor XIII und/oder Fibrinogen.

9. Methode entsprechend der Ansprüche 1, 2 sowie 5 bis 7, wobei als Faktor XIII-Inhibitoren poly- oder monoklonale Antikörper eingesetzt werden.

10. Methode entsprechend der Ansprüche 1, 2 sowie 5 bis 7, wobei als Faktor XIII-Inhibitoren peptidische Inhibitoren eingesetzt werden.

11. Methode entsprechend der Ansprüche 1, 2 sowie 5 bis 7, wobei als Faktor XIII-Inhibitoren niedermolekulare Inhibitoren eingesetzt werden.

12. Methode entsprechend Anspruch 1 sowie 5 bis 8, wobei als Fibrinogen-Aktivatoren Proteasen, die gegenüber Faktor XIII nicht aktiv sind, eingesetzt werden.

13. Methode entsprechend Anspruch 1, sowie 5 bis 8, wobei als Fibrinogen Aktivator Batroxobin eingesetzt wird.

14. Methode gemäß Ansprüchen 6 bis 7 wobei als Inhibitor des thrombolytischen Systems Aprotinin eingesetzt wird.

## Claims

1. Method of differentiating between factor XIII deficiency states and fibrinogen deficiency states by simultaneously determining the two potential deficiency states in a sample and comparing them
firstly
- by comparing specific TEG parameters in the presence with specific TEG parameters in the absence of factor XIII inhibitors
or
- by comparing specific TEG parameters with addition of factor XIII with those without addition of factor XIII,
and secondly
- by comparing specific TEG parameters in the presence of fibrinogen activators with specific TEG parameters in the presence of activators of fibrinogen in normal blood or plasmas.

2. Method according to Claim 1, where a determination of factor XIII deficiency states is made by measuring, evaluating and comparing specific TEG parameters in the presence and in the absence of factor XIII inhibitors.

3. Method according to Claim 1, where a determination of factor XIII deficiency states is made by measuring, evaluating and comparing specific TEG parameters with addition of factor XIII and without addition of factor XIII.

4. Method according to Claim 1, where a determination of fibrinogen deficiency states by comparing thrombelastographic parameters in the presence of fibrinogen activators with normal TEG values obtained by using fibrinogen activators in normal blood or plasmas, where the fibrinogen activators are proteases which activate fibrinogen while not being reactive with factor XIII.

5. Method according to Claim 1 for differentiating between factor XIII deficiency states and fibrinogen deficiency states with the exclusion of the effect of platelets by determining the TEG parameters when using platelet-poor plasma or by using platelet antagonists when using whole blood or platelet-rich plasma.

6. Method according to Claim 1 for differentiating between factor XIII deficiency states and fibrinogen deficiency states with the exclusion of the effect of the thrombolytic system by determining the TEG parameters when using inhibitors of the thrombolytic system.

7. Method according to Claim 1 for differentiating between factor XIII deficiency states and fibrinogen deficiency states when using platelet-poor plasma or by using platelet antagonists when using whole blood or platelet-rich plasma and with simultaneously inhibiting the thrombolytic system according to Claims 5 and 6.

8. Use of a method according to Claims 1 to 7 as the basis for decision-making on a tailored substitution of factor XIII and/or fibrinogen.

9. Method according to Claims 1, 2 and 5 to 7, where polyclonal or monoclonal antibodies are employed as factor XIII inhibitors.

10. Method according to Claims 1, 2 and 5 to 7, where peptidic inhibitors are employed as factor XIII inhibitors.

11. Method according to Claims 1, 2 and 5 to 7, where low-molecular-weight inhibitors are employed as factor XIII inhibitors.

12. Method according to Claim 1 and 5 to 8, where proteases which are not active against factor XIII are employed as the fibrinogen activators.

13. Method according to Claim 1 and 5 to 8, where batroxobin is employed as the fibrinogen activator.

14. Method according to Claims 6 to 7, where aprotinin is employed as the inhibitor of the thrombolytic system.

## Revendications

1. Procédé pour distinguer des états de déficit en facteur XIII vis-à-vis d'états de déficit en fibrinogène par détermination simultanée des deux états de déficit potentiels à partir d'un échantillon ainsi que leur comparaison,
d'une part,
- par comparaison de certains paramètres TEG en présence d'inhibiteurs du facteur XIII avec certains paramètres TEG en absence de ces inhibiteurs
ou
- par comparaison de certains paramètres TEG avec addition de ces inhibiteurs sans addition de facteur XIII
ainsi que d'autre part
- par comparaison de certains paramètres TEG en présence d'activateurs du fibrinogène, avec certains paramètres TEG en présence d'activateurs du fibrinogène dans du sang normal ou des plasmas normaux.

2. Procédé selon la revendication 1, dans lequel s'effectue une détermination d'états de déficit en facteur XIII par mesure, évaluation et comparaison de certains paramètres TEG en présence et en absence d'inhibiteurs du facteur XIII.

3. Procédé selon la revendication 1, dans lequel s'effectue une détermination d'états de déficit en facteur XIII par mesure, évaluation et comparaison de certains paramètres TEG avec addition de facteur XIII et sans addition de facteur XIII.

4. Procédé selon la revendication 1, dans lequel s'effectue une détermination d'états de déficit en fibrinogène par comparaison de paramètres thromboélastographiques en présence d'activateurs du fibrinogène avec des valeurs TEG normales obtenues par utilisation d'activateurs du fibrinogène dans du sang normal ou des plasmas normaux, les activateurs du fibrinogène étant des protéases qui activent le fibrinogène, mais ne sont pas réactives vis-à-vis du facteur XIII.

5. Procédé selon la revendication 1 pour distinguer des états de déficit en facteur XIII vis-à-vis d'états de déficit en fibrinogène avec exclusion de l'effet des plaquettes, par détermination des paramètres TEG en utilisant du plasma pauvre en plaquettes ou en utilisant des antagonistes des plaquettes lorsqu'on utilise du sang entier ou du plasma riche en plaquettes.

6. Procédé selon la revendication 1 pour distinguer des états de déficit en facteur XIII vis-à-vis d'états de déficit en fibrinogène avec exclusion des effets du système thrombolytique, par détermination des paramètres TEG en utilisant des inhibiteurs du système thrombolytique.

7. Procédé selon la revendication 1 pour distinguer des états de déficit en facteur XIII vis-à-vis d'états de déficit en fibrinogène en utilisant du plasma pauvre en plaquettes ou en utilisant des antagonistes des plaquettes lorsqu'on utilise du sang entier ou du plasma riche en plaquettes, ainsi qu'avec inhibition simultanée du système thrombolytique selon les revendications 5 et 6.

8. Utilisation d'un procédé selon les revendications 1 à 7 comme base pour la prise de décision sur une substitution ciblée de facteur XIII et/ou de fibrinogène.

9. Procédé conforme aux revendications 1, 2 ainsi que 5 à 7, dans lequel on utilise comme inhibiteurs du facteur XIII des anticorps monoclonaux ou polyclonaux.

10. Procédé conforme aux revendications 1, 2 ainsi que 5 à 7, dans lequel on utilise comme inhibiteurs du facteur XIII des inhibiteurs peptidiques.

11. Procédé conforme aux revendications 1, 2 ainsi que 5 à 7, dans lequel on utilise comme inhibiteurs du facteur XIII des inhibiteurs de faible masse moléculaire.

12. Procédé conforme à la revendication 1 ainsi qu'aux revendications 5 à 8, dans lequel on utilise comme activateurs du fibrinogène des protéases qui ne sont pas actives vis-à-vis du facteur XIII.

13. Procédé conforme à la revendication 1 ainsi qu'aux revendications 5 à 8, dans lequel on utilise comme activateur du fibrinogène la batroxobine.

14. Procédé selon les revendications 6 et 7, dans lequel on utilise comme inhibiteur du système thrombolytique l'aprotinine.
